# EUROPÄISCHE PATENTANMELDUNG

(11) **EP 0 588 229 A2**
(43) Veröffentlichungstag der Anmeldung: **23.03.1994**
(21) Anmeldenummer: 93114414.1
(22) Anmeldetag: 08.09.1993
(51) Int. Cl.: C07F 9/6558, C07D 257/02, C07D 401/12, A61K 49/02, A61K 47/48

(54) **Makrozyklische Chelatbildner zur Herstellung von Technetium- oder Rheniumkomplexen**

(30) Priorität: 12.09.1992 DE 4230612
(71) Anmelder: CIS BIO INTERNATIONAL, F-91000 Saclay (FR)
(72) Erfinder: Stahl, Wilhelm, Dr., D-65929 Frankfurt am Main (DE); Walch, Axel, Dr., D-60487 Frankfurt am Main (DE); Doll, Wilfried, D-64569 Nauheim (DE); Kuhlmann, Ludwig, Dr., D-65439 Flörsheim am Main (DE); Hachmann, Henning, Dr., D-60529 Frankfurt am Main (DE); Steinsträsser, Axel, Dr. Dr., D-65835 Liederbach (DE)
(74) Vertreter: Dubois-Chabert, Guy

(57) **Zusammenfassung**

Die Erfindung betrifft einen makrozyklischen Chelatbildner der allgemeinen Formel (I) und ihre Verwendung zur Markierung von Substanzen insbesondere mit radioaktiven Technetium- oder Rheniumisotopen, sowie die Verwendung der Chelate in Testkits zum Nachweis von Tumoren.

## Beschreibung

Die Erfindung betrifft makrozyklische Chelatbildner, ihre Verwendung zur Markierung von Substanzen mit insbesondere radioaktiven Technetium- oder Rheniumisotopen, sowie die Verwendung der Chelate in diagnostischen und therapeutischen Verfahren.

In den vergangenen Jahren wuchs das Verlangen, chemische Verbindungen mit radioaktiven Nukliden zu markieren. Vor allem im Bereich der medizinischen Diagnostik, wo pathologische Zustände bereits durch Substanzen angezeigt werden können, die im Organismus nur in ppm oder gar in noch geringeren Konzentrationen vorkommen, kann man heute nicht mehr auf radioaktiv markierte Substanzen verzichten.

Insbesondere Technetium-99m ist wegen seiner günstigen physikalischen Eigenschaften (Fehlen einer Korpuskularstrahlung, γ-Energie von 140 keV und Halbwertszeit von 6 Stunden) und der damit verbundenen geringen Strahlenbelastung zum wichtigsten Radionuklid in der nuklearmedizinischen Diagnostik geworden.

Technetium-99m, das sich aus Nuklidgeneratoren gewinnen läßt, liegt zunächst als Pertechnetat vor, das in dieser Form, z. B. für die Schilddrüsen- und Hirnszintigraphie geeignet ist. Die Szintigraphie anderer Organe mittels Technetium-99m gelingt mit Hilfe bestimmter "Transportsubstanzen", die einerseits in der Lage sind, Technetium zu binden und andererseits das Radionuklid im Zielorgan mit hoher Selektivität anzureichern. Zur Markierung der organspezifischen "Transportsubstanz" mit Technetium-99m muß das aus dem Nuklidgenerator eluierte Pertechnetat zuerst in eine niedrigere Oxidationsstufe überführt werden. In dieser reduzierten Form bildet Technetium mit der organspezifischen Substanz mehr oder weniger stabile Verbindungen. Für die Knochenszintigraphie werden z. B. Tc-99m-Phosphonsäure-Derivate, vor allem organische Phosphonsäuren, eingesetzt. So liegt in der im Europäischen Patent 2485 beschriebenen Markierungseinheit das Natriumsalz der 3,3-Diphosphono-1,2-propandicarbonsäure als organspezifische "Transportsubstanz" vor. In dem Europäischen Patent 108253 werden Tc-99m-Tri- und Tetraphosphonsäuren zur szintigraphischen Darstellung des Retikulo-endothelialen Systems (RES), insbesondere der Leber, beschrieben. Der Tc-99m-Komplex mit Diethylentriaminpentaessigsäure (DTPA) findet zur Diagnostik von Nierenerkrankungen bzw. pathologischen Hirnprozessen Anwendung.

Zur Markierung bestimmter Substanzen mit Technetium-99m und zur Herstellung von für den klinischen Routinebedarf geeigneten Testkits wurden spezielle Verfahren entwickelt und beschrieben. Zur Präparierung von Markierungsbestecken für biologisch interessante Makromoleküle, insbesondere Porphyrine, Dextrane, Cytochrome und Myoglobin, wird eine Methode beschrieben (G. D. Zanelli, D. Ellison, M. P. Barrowcliffe, Nucl. Med. Commun. 8, 199 bis 206, 1987), bei der die zu markierende Substanz zusammen mit p-Aminobenzoesäure und einer salzsauren SnCl₂-Lösung lyophilisiert wird. Zur Rekonstitution und Markierung dieses Kits gibt man Tc-99m-Generatoreluat, das vorher mit genügend Pufferlösung, z. B. Citrat-Natriumchlorid-Puffer pH 9,5, verdünnt wurde, hinzu. Für säureempfindliche Substanzen ist diese Methode jedoch nicht geeignet.

Bei einem anderen Verfahren (E. K. J. Pauweis, R. I. J. Feitsma, internationale Patentanmeldung WO 86/03010) wird durch vierstündiges Erhitzen in stark salzsaurer Lösung auf 140°C Tc-99m-Pertechnetat zuerst reduziert und an einer Verbindung, die eine Aminogruppe enthält, z. B. Dimethylformamid, gebunden. Das reaktionsfähige Tc-99m-markierte Zwischenprodukt, das als schwerlösliche kristalline Substanz ausfällt, wird in einer Pufferlösung, z. B. Natriumcarbonatlösung, durch einstündige Inkubation bei Raumtemperatur mit der zu markierenden Verbindung umgesetzt. Die Methode arbeitet zwar zinnfrei, ist aber wegen der aufwendigen Verfahrensschritte für die Routineanwendung kaum geeignet.

Zur Markierung von Proteinen, insbesondere Antikörpern, kennt man zwei verschiedene Wege. Bei der direkten Methode wird das reduzierte Technetium-99m durch Donorgruppen (Amino-, Amid-, Thiol- etc.) des Proteins gebunden.

Solche Verfahren sind in dem Europäischen Patent 5638 und dem US-Patent 4.478.815 beschrieben. Dort werden Zinn-II-Salze im Überschuß zur gleichzeitigen reduktiven Spaltung von Disulfid-Brücken und zur Reduktion des zugesetzten Tc-99m-Pertechnetats benutzt. Im allgemeinen benötigt man zur Spaltung der -S-S-Bindung längere Inkubationszeiten (24 Stunden), wobei F(ab')₂-Fragmente partiell zu Fab'-Fragmenten gespalten werden. Neuere Literaturangaben (z. B. Journal of Nuclear Medicine 27 (1986), Seite 685 bis 693 und 1315 bis 1320 sowie International Journal of Nuclear Medicine Biology 12 (1985) Seiten 3 bis 8) zeigen, daß das Verhältnis der beiden Fragmente abhängig ist von der "Bezinnungsreaktion" und daß sich das Verhältnis der beiden Komponenten nach der Tc-99m-Markierung nicht mehr nennenswert ändert, wobei die Hauptkomponente Tc-99m-markiertes F(ab') ist. In allen Fällen mußte das markierte F(ab')-Fragment nachgereinigt werden, da trotz mindestens 30-minütiger Reaktionszeit keine quantitative Umsetzung des Pertechnetats erreicht wurde.

Bei einer schnellen chemischen Methode zur Tc-99m-Markierung humaner Plasmaproteine (D. W. Wong, F. Mishkin, T. Lee, J. Nucl. Med. 20, 967 bis 972, 1979) wird Pertechnetat zuerst in saurer Lösung durch Zinn-II-Ionen reduziert und das reduzierte Technetium dann mit dem Protein umgesetzt.

Unter Zuhilfenahme von bifunktionalen Komplexbildnern läßt sich eine stabile Markierung von Substanzen mit Radioisotopen erreichen. In dem US-Patent 4.479.930 werden die cyclischen Anhydride von DTPA und EDTA als Chelatisierungsmittel nicht nur für In-111 und Ga-67, sondern auch für Tc-99m angeführt. In dem europäischen Patent 35765 wird die Verwendung von Deferoxamin als Komplexierungsagens für Technetium-99m an Proteine erwähnt. In der internationalen Patentanmeldung WO 85/3063 werden die partiell reduzierten Disulfid-Brücken im Antikörper mit dem Natriumsalz des Tetrachlornitridotechnetats, das durch Reaktion von Pertechnetat mit Natriumazid vorher präpariert werden muß, umgesetzt. In der europäischen Patentanmeldung 194853 benutzt man ebenfalls durch Reduktion in Antikörperfragmenten erzeugte freie Thiolgruppen zur Bindung von [(7-Maleimidoheptyl)imino-bis(ethylennitrilo)]-tetraessigsäure als Chelatkomplex. Die Kupplung des Komplexes an den Antikörper erfolgt über die Reaktion der SH-Gruppen mit der Doppelbindung im Maleinimidteil der Komplexverbindung, während das radioaktive Metallion über die Nitrilodiessigsäure-Reste komplexiert wird.

Metallothionein, ein metallbindendes Protein mit einem Molekulargewicht von 6000 und einem hohen Cysteinanteil im Molekül wurde als Komplexbildner in Antikörper eingeführt (G. L. Toman, R. J. Hadjian, M. M. Morelock et al, J. Nucl. Med. 25, 20, 1984). Durch Austausch mit Tc-99m-Glucoheptonat ließ sich das Antikörper-Metallothionein-Konjugat mit Technetium markieren. Der Austausch blieb jedoch unvollständig, so daß eine Nachreinigung erforderlich war. Mehrere Bisthiosemicarbazon-Liganden wurden ebenfalls als bifunktionelle Chelatbildner beschrieben (Y. Arano, A. Yokoyama, H. Magat et al., Int. J. Nucl. Med. Biol. 12, 425 bis 430, 1986). p-Carboxyethylphenylglyoxal-di(N-methylthiosemicarbazon) wurde mit humanem Serumalbumin konjugiert. Der Tc-99m-markierte 1 : 1-Komplex zeigte eine gewisse Instabilität, während Komplexe mit einem höheren Verhältnis als 1 : 1 eine vermehrte Leberspeicherung aufwiesen. Die Kopplung eines Diamid-dimercaptid-N₂S₂-Liganden an Proteine (A. R. Fritzberg, S. Kasina, J. M. Reno et al., J. Nucl. Med. 27, 957 bis 958, 1986) erfolgt über eine zusätzliche funktionelle Gruppe. So wurde z. B. 4,5-Di(S-ethylcarbonylmercaptoacetamid)-pentanoyl-N-hydroxysuccinimid mit einem anti-Melanomantikörper umgesetzt. Das entstandene Konjugat wurde bei pH 8 und 50°C mit Tc-99m-Tartratlösung inkubiert. Nach einer Stunde waren 78 % des Technetiums vom Tartrat auf den Antikörper übertragen.

Um Technetium-99m diagnostisch breit nutzen zu können, ist es notwendig, dieses Nuklid in das zu untersuchende Organ selektiv zu transportieren. Aus anderen Organen oder Organsystemen sollte das Technetium-99m wieder schnell eliminiert oder überhaupt nicht hingebracht werden, um jede unnötige Strahlenbelastung für den Patienten zu vermeiden. Zu diesem Zweck werden bisher vorwiegend Substanzen eingesetzt` die direkt mit Technetium-99m markierbar sind und eine hohe Organspezifität aufweisen. Darüber hinaus gibt es jedoch eine Reihe von Substanzen, die zwar eine hohe Organspezifität aufweisen, aber nicht direkt markierbar sind. Dies können Proteine (Fibrinogen, Humanserumalbumin), Enzyme (Streptokinase, Lactatdehydrodgenase), Zucker (Dextran, Glucose) oder auch Polymere sein. Dazu gehören ebenfalls niedermolekulare Substanzen wie etwa Fettsäuren, die sich durch den hohen Energiebedarf des Herzens im Myocardgewebe anreichern. Um diese Substanzen markieren zu können, werden sie mit Komplexbildnern gekoppelt, die ihrerseits Technetium-99m fest binden können.

Als geeignete Komplexbildner für die Komplexierung von Metallionen sind makrozyklische Amine, u. a. auch Cyclame, bekannt. Die Komplexierungsausbeute liegt für den Technetium-Cyclam Komplex unter geeigneten Bedingungen bei 99 %. Einzelheiten über Technetium-Aminkomplexe sind in D. E. Troutner, J. Simon, A. R. Ketrin, W. A. Volkert, R. A. Holmes, J. Nucl. Med. 21 (1980), 443 oder S. A. Zuckmann, G. M. Freeman, D. E. Troutner, W. A. Volkert, R. A. Holmes, D. G. van der Keer, E. K. Barefiled. Inorg. Ch. 20 (1981), 3386 oder J. Simon, D. Troutner, W. A. Volkert, R. A. Holmes, Radiochem. Radioanal. Lett. 47 (1981), 111 erwähnt. Auch substituierte Cyclame sind, sowohl am 1-Stickstoff, als auch am 6-Kohlenstoff substituiert, bekannt (A. R. Ketrin, D. E. Troutner et al., Int. J. Nucl. Med. Biol. 11 (1984), 113 oder J. Simon. Diss. Abstr. Int. B42 (1981), 645 oder M. Struden, T. A. Kaden, Helv. Chim. Acta 69 (1986), 2081 oder E. Kimura, R. Machida, M. Kodama, J. Am. Chem. Soc. 106 (1984), 5497.

Eine Reihe von Versuchen, Amin- und auch andere Liganden an Proteine zu konjugieren (s. Fritzberg et al., J. Nucl. Med. 27 (1986), 957 oder Tolman et al., J. Nucl. Med. 25 (1984), 20 oder Arano et al., Int. J. Nucl. Med. Biol. 12 (1986) 425) führten zu Produkten, die die hohen in vivo Stabilitätsansprüche nicht oder nur teilweise erfüllten.

Mit bestimmten Metallisotopen, wie z. B. Indium-111 (Europäische Patentanmeldung 279 307) oder Rhodium-105 bzw. -101 (Europäische Patentanmeldung 296 522) war es jedoch bereits möglich, relativ stabile Chelatkomplexe zu erzeugen. Höherwertige Metallionen, wie Technetium und Rhenium bilden mit den bisher bekannten Chelatbildnern allerdings keine ebenso stabilen Komplexe.

Obwohl die in der Europäischen Patentschrift 304 780 beschriebenen Technetiumchelatkomplexe den Forderungen nach einer hohen Stabilität bereits näherkamen, war eine diesbezügliche Verbesserung dennoch wünschenswert.

Der vorliegenden Erfindung lag daher die Aufgabe zugrunde, zur Herstellung äußerst stabiler, insbesondere radioaktiver, Technetium- und Rheniumkomplexe geeignete Chelatbildner zur Verfügung zu stellen, die auf einfache Weise herstellbar und für die Markierung von Substanzen einsetzbar sind.

Die Lösung dieser Aufgabe gelang überraschenderweise mit substituierten makrozyklischen Aminen der allgemeinen Formel (I) 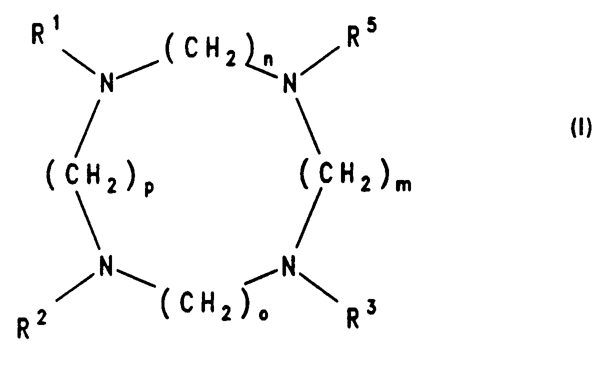
worin
- R¹, R² und R³: gleich oder verschieden sind und Wasserstoff oder -CH₂-PO₂HR⁴ und
- m, n, o, p: gleich oder verschieden sind und 1, 2, 3 oder 4 bedeuten und
- R⁴: Hydroxy, Alkoxy mit 1 bis 4 C-Atomen oder Phenoxy, welches gegebenenfalls mit C₁- bis C₄-Alkyl substituiert ist
und
- R⁵: eine Seitenkette der Formel (II) ist,

[-X-(Y)_{q}]ᵣ-(Z)ₛ-R⁶ (II)

wobei
- q: für 0 oder 1 und
- r: für 1, 2, 3, 4 oder 5 und
- s: für 0 oder 1 steht, mit der Maßgabe, daß s nur 1 sein kann, wenn
- q: = 1
und
- X, Z: gleich oder verschieden sind und Alkylen mit 1 bis 40 C-Atomen, ortho-, meta- oder para-Aralkylen mit 7 bis 40 C-Atomen, welches über den Alkylrest am Stickstoff und über den Arylrest oder einen weiteren am Arylrest gebundenen Alkylrest an R⁶ gebunden ist, bedeuten und
- Y: einen Substituenten einer der folgenden Formeln bedeutet, 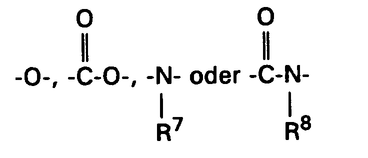 wobei
- R⁷: Wasserstoff, Methyl, Ethyl, Benzyl, Acetyl oder Benzoyl und
- R⁸: Wasserstoff, Methyl, Ethyl oder Benzyl bedeuten und
- R⁶: eine Gruppe einer der folgenden Formeln bedeuten

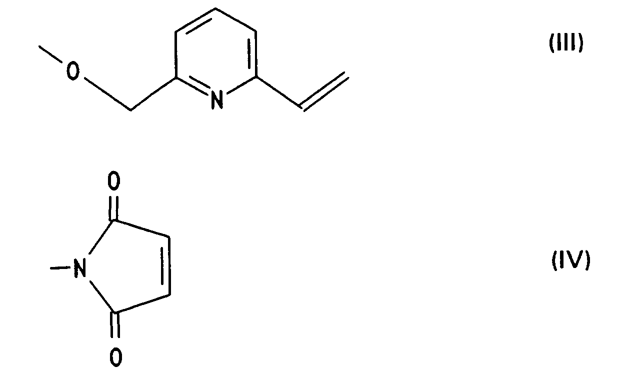

-N=C=S (V)

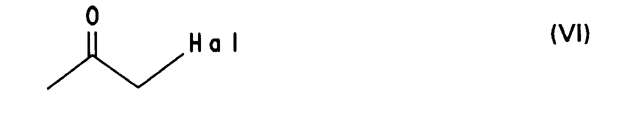

oder
-N₃, -Hal oder -SH,
wobei Hal für Fluor, Chlor, Brom oder Iod steht.

Insbesondere betrifft die Erfindung Verbindungen gemäß Formel (I), worin
- R¹, R² und R³: gleich sind und -CH₂-PO₂HR⁴ bedeuten und
- m, n, o, p: gleich oder verschieden sind und 2,3 oder 4 bedeuten und
- R⁴: Hydroxy, Alkoxy mit 1 bis 4 C-Atomen oder Phenoxy, welches gegebenenfalls mit C₁- bis C₄-Alkyl substituiert ist, bedeutet und
- R⁵: eine Seitenkette der Formel (II) ist,

[-X-(Y)_{q}]ᵣ-(Z)ₛ-R⁶ (II)

wobei
- q: für 0 oder 1 und
- r: für 1, 2, 3, 4 oder 5 und
- s: für 0 oder 1 steht, mit der Maßgabe, daß S nur 1 sein kann, wenn
- q: = 1
und
- X, Z: gleich oder verschieden sind und Alkylen mit 1 bis 20 C-Atomen, ortho-, meta- oder para-Aralkylen mit 7 bis 20 C-Atomen, welches über den Alkylrest am Stickstoff und über den Arylrest oder einen weiteren am Arylrest gebundenen Alkylrest an R⁶ gebunden ist, bedeuten und
- Y: einen Substituenten einer der folgenden Formeln bedeutet, 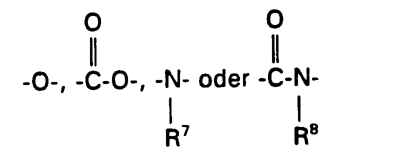 wobei
- R⁷: Wasserstoff, Methyl, Ethyl, Benzyl, Acetyl oder Benzoyl und
- R⁸: Wasserstoff, Methyl, Ethyl oder Benzyl bedeuten und
- R⁶: eine Gruppe einer der folgenden Formeln bedeuten

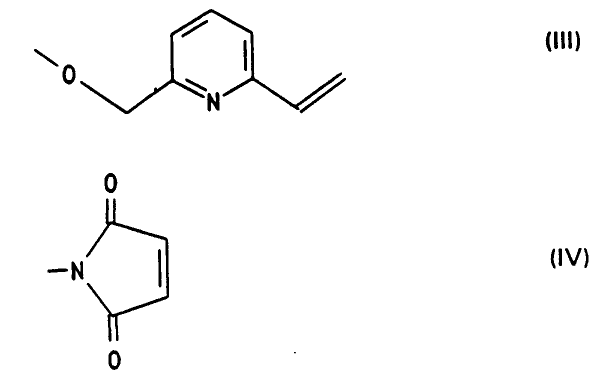

-N=C=S (V)

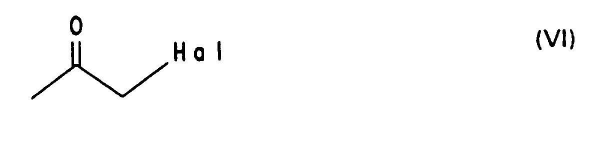

oder
-N₃, Hal oder -SH,
wobei Hal für Fluor, Chlor, Brom oder Iod steht.

Weiterhin bevorzugt sind Verbindungen gemäß Formel (I), worin
- R¹, R² und R³: gleich sind und -CH₂-PO₂HR⁴ bedeuten und
- m, n, o, p: gleich oder verschieden sind und 2 oder 3 bedeuten und
- R⁴: Hydroxy, Alkoxy mit 1 bis 4 C-Atomen oder Phenoxy, welches gegebenenfalls mit C₁- bis C₄-Alkyl substituiert ist, bedeutet und
- R⁵: eine Seitenkette der Formel (II) ist,

[-X-(Y)_{q}]ᵣ-(Z)ₛ-R⁶ (II)

wobei
- q: für 0 oder 1 und
- r: für 1, 2, 3, 4 oder 5 und
- s: für 0 oder 1 steht, mit der Maßgabe, daß S nur 1 sein kann, wenn
- q: = 1
und
- X, Z: gleich oder verschieden sind und Alkylen mit 1 bis 20 C-Atomen, ortho-, meta- oder para-Aralkylen mit 7 bis 20 C-Atomen, welches über den Alkylrest am Stickstoff und über den Arylrest oder einen weiteren am Arylrest gebundenen Alkylrest an R⁶ gebunden ist, bedeuten und
- Y: einen Substituenten einer der folgenden Formeln bedeutet, 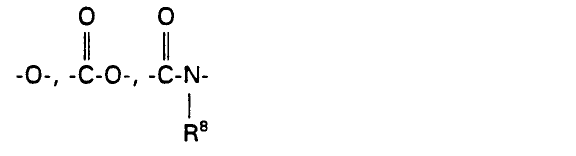 worin
- R⁸: für Wasserstoff, Methyl, Ethyl oder Benzyl steht, und
- R⁶: eine Gruppe einer der folgenden Formeln bedeutet

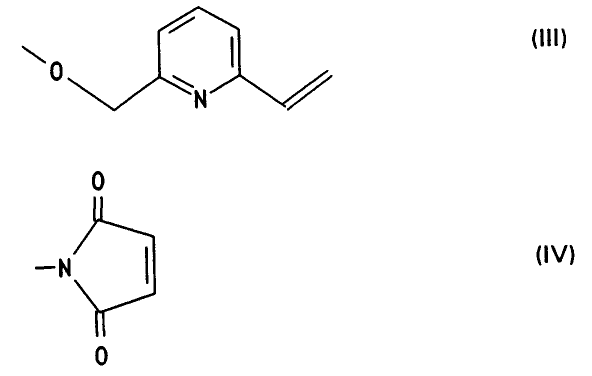

-N=C=S (V)

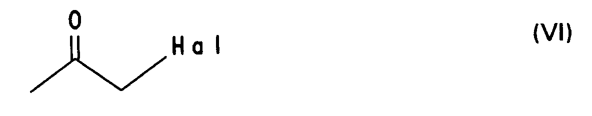

wobei Hal für Fluor, Chlor, Brom oder Iod steht oder-N₃.

Besonders bevorzugt sind Verbindungen gemäß Formel (I), worin
- R¹, R² und R³: gleich sind und -CH₂-PO₂HR⁴ bedeuten und
- m, n, o, p: gleich oder verschieden sind und 2 oder 3 bedeuten und
- R⁴: Hydroxy, Alkoxy mit 1 bis 4 C-Atomen oder Phenoxy, welches gegebenenfalls mit C₁- bis C₄-Alkyl substituiert ist bedeutet und
- R⁵: eine Seitenkette der Formel (II) ist,

[-X-(Y)_{q}]ᵣ-(Z)ₛ-R⁶ (II)

wobei
- q: für 0 oder 1 und
- r: für 1, 2, 3, 4 oder 5 und
- s: für 0 oder 1 steht, mit der Maßgabe, daß S nur 1 sein kann, wenn
- q: = 1
und
- X, Z: gleich oder verschieden sind und Alkylen mit 1 bis 20 C-Atomen, ortho-, meta- oder para-Aralkylen mit 7 bis 20 C-Atomen, welches über den Alkylrest am Stickstoff und über den Arylrest oder einen weiteren am Arylrest gebundenen Alkylrest an R⁶ gebunden ist, bedeuten und
- Y: für -O-
und
- R⁶: für eine Gruppe einer der folgenden Formeln steht 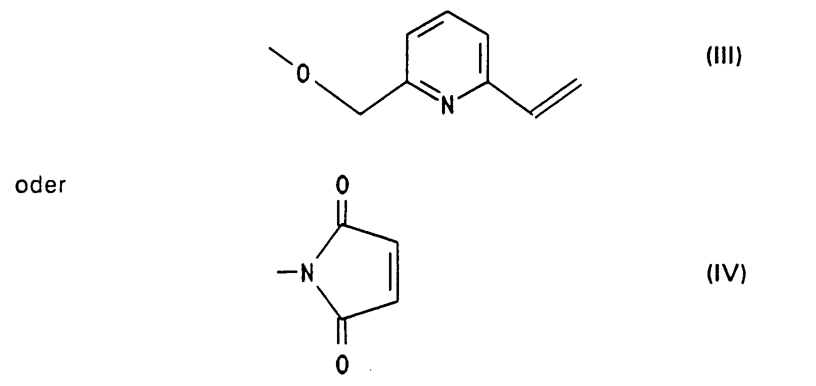
Ganz besonders bevorzugt sind schließlich diejenigen Verbindungen gemäß Formel (I),
worin
- R¹, R² und R³: gleich sind und -CH₂-PO₂HR⁴ bedeuten und
- m, n, o, p: gleich oder verschieden sind und 2 oder 3 bedeuten und
- R⁴: Hydroxy, Alkoxy mit 1 bis 4 C-Atomen oder Phenoxy, welches gegebenenfalls mit C₁- bis C₄-Alkyl substituiert ist, bedeutet und
- R⁵: eine Seitenkette der Formel (II) ist,

[-X-(Y)_{q}]ᵣ-(Z)ₛ-R⁶ (II)

wobei
- q: für 0 oder 1 und
- r: für 1, 2, 3, 4 oder 5 und
- s: für 0 oder 1 steht, mit der Maßgabe, daß S nur 1 sein kann, wenn
- q: = 1
und
- X, Z: gleich oder verschieden sind und Alkylen mit 1 bis 20 C-Atomen, ortho-, meta- oder para-Aralkylen mit 7 bis 20 C-Atomen, welches über den Alkylrest am Stickstoff und über den Arylrest oder einen weiteren am Arylrest gebundenen Alkylrest an R⁶ gebunden ist, bedeuten und
- Y: für -O-und
- R⁶: eine Gruppe der folgenden Formel steht

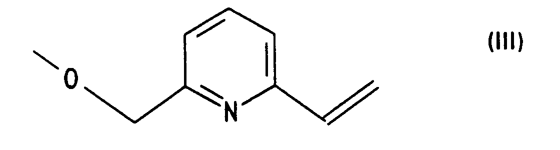

Unter Aryl werden in obiger Beschreibung bevorzugt Phenyl und Naphthyl, insbesondere bevorzugt Phenyl verstanden. Alkylgruppen mit mehr als 2 C-Atomen können sowohl geradkettig als auch verzweigt sein. Bei der Alkylengruppe X handelt es sich bevorzugt um geradkettige Alkylene mit bis zu 40 C-Atomen.

Hervorgehoben werden soll auch eine Verbindung der Formel (VII), die eine insbesondere bevorzugte Ausführungsform der vorliegenden Erfindung darstellt 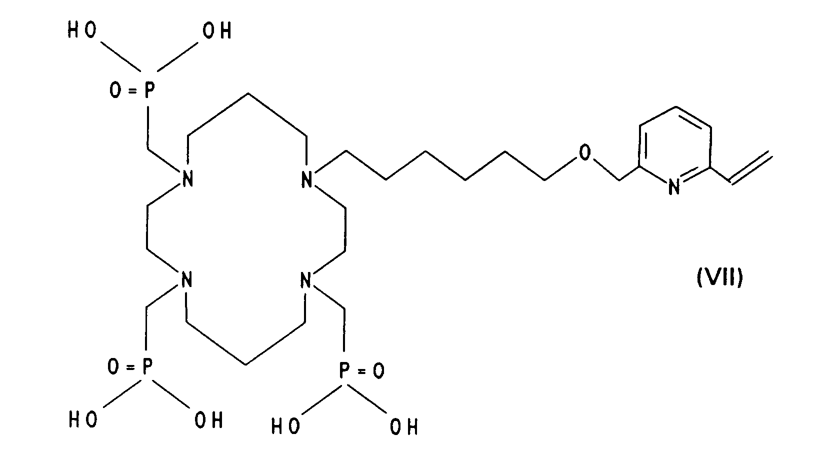
Die vorliegende Erfindung betrifft weiterhin einen Chelatkomplex, enthaltend eine Verbindung der Formel (I) und ein Technetium- oder Rheniumion.

Die vorliegende Erfindung betrifft weiterhin ein Konjugat enthaltend einen Chelatkomplex wie oben beschrieben und, über die Seitenkette R⁵ gebunden, eine Substanz von diagnostischem und/oder therapeutischem Interesse. Unter "Substanzen von diagnostischem und/oder therapeutischem Interesse" werden in erster Linie solche Verbindungen verstanden, die in der medizinischen Diagnostik als "Transportsubstanzen" eingesetzt werden können, also meist organspezifische Substanzen, wie Antikörper, Antikörperfragmente [z. B. F(ab')₂- oder Fab'-Fragmente], Proteine (z. B. Fibrinogen), Peptide (z. B. Somatostatin), Oligonukleotide, Zucker (z. B. Dextran, Glucose) oder auch Polymere. Andererseits können aber auch generell solche Substanzen mit den erfindungsgemäßen makrozyklischen Chelatbildnern bzw. daraus hergestellten Chelatkomplexen markiert werden, die mit deren funktioneller Gruppe an der Seitenkette unter Ausbildung einer chemischen Bindung reagieren. Gedacht ist hierbei z. B. an die "Überwachung" von chemischen Substanzen in Produktionsanlagen, die Bestimmung ihrer Konzentration, Strömungsgeschwindigkeit und Verweilzeit.

Die vorliegende Erfindung betrifft weiterhin Verfahren zur Herstellung von mit Technetium- oder Rheniumisotopen markierten makrozyklischen Aminen sowie Konjugaten enthaltend solche Chelatkomplexe sowie Substanzen von diagnostischem und/oder therapeutischem Interesse.

Die Herstellung der erfindungsgemäßen N-monoalkylierte Makrocyclen erfolgt ausgehend von käuflichen Azamakrozyklen (z. B.: 1,4,7,10-Tetraazacyclododecantetrahydrochlorid, 1,4,8,11-Tetraazacyclotetradecan, 1,5,8,12-Tetraazacyclopentadecan, Vertrieb: Strem Chemicals GmbH, Querstr. 2, 7640 Kehl, Deutschland) entsprechend der in den üblichen Lehrbüchern (z. B.: Advanced Organic Chemistry, 3. Edition, J. March, John Wiley & Sons, New York, 1985) beschriebenen Verfahren zur Alkylierung von Aminen durch Umsetzung mit einem Alkylhalogenid, wie z. B. Alkylbromid in Gegenwart basischer Mittel, wie z. B. Kaliumhydroxid oder Natriumhydroxid. Dabei sorgt ein Überschuß des Makrozyklus für die überwiegende Entstehung von monoalkyliertem Cyclam. Der verwendete Überschuß kann durch Kristallisation zum großen Teil zurückgewonnen werden. Die für die Anknüpfung an Antikörper oder andere diagnostisch oder therapeutisch anwendbare Substanzen nötige Kopplungsgruppe kann je nach ihrer Reaktivität bereits Bestandteil des Alkylbromids sein oder später eingeführt werden. Für den Fall der Verwendung hochreaktiver Gruppen (z. B. Isothiocyanat) ist es zweckmäßig an ihrer Stelle zunächst eine leicht substituierbare Einheit einzuführen (z. B. Bromid) und diese am Ende der Synthese analog einem literaturbekannten Verfahren (z. B.: Advanced Organic Chemistry, 3. Edition, J. March, John Wiley & Sons; New York, 1985) gegen die gewünschte Kopplungsgruppe auszutauschen. Die Einführung der Methylphosphonsäuregruppen unterscheidet sich von der Einführung strukturell verwandter Methylcarbonsäuren (I. M. Helps et al., Tetrahedron 1989, 45, 219). Da die entsprechenden halogenierten Derivate wie etwa Iodmethanphosphonsäureester nur eine unzureichende Reaktivität aufweisen, empfiehlt sich hier die Anwendung einer Mannich-Reaktion analog zu den Verfahren von Fields und Jagodic (E. K. Fields, J. Am. Chem. Soc. 1952, 74, 1528; V. Jagodic, Chem. Ber. 1960, 2308). Die erfindungsgemäße Reaktion gelingt nur, wenn der entsprechende Makrocyclus in einem aprotisch dipolaren Lösungsmittel, wie z. B. Acetonitril, Proprionitril oder Dimethylacetamid unter Ausschluß von Wasser mit Paraformaldehyd und Diethylphosphit umgesetzt wird. Der Wasserausschluß verhindert hier die Entstehung von Hydroxymethylphosphonsäurediethylester, was nur zu einem vorzeitigen Verbrauch der Reagenzien führen würde (analog zu C. J. Broan et al. Synthesis 1992, 63). Unter dieser Reaktionsbedingung entstand das gewünschte Produkt jedoch nur in 3 % Ausbeute. Als Hauptprodukt entstand ein Aminal, ein durch eine Methylengruppe überbrückter Macrocyclus. (Dies wurde schon für verwandte Verbindungen beschrieben: C. J. Broan et al. Synthesis 1992, 63 und darin zitierte Literatur.) Die Existenz des Aminals konnte anhand der Synthese der folgenden Modellverbindung (VIII) nachgewiesen werden: 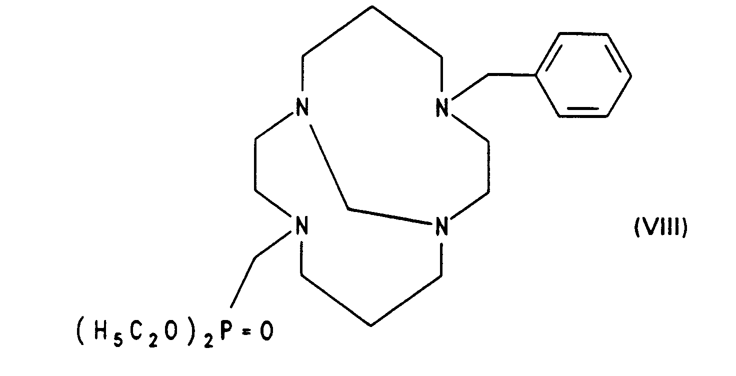
Molmasse: 452 (durch Massenspektrometrie)
¹H-NMR: 100 MHz (Cl₃CD): 3,59 ppm; 300 MHz (Cl₃CD): 3,62 ppm
Überraschenderweise läßt sich die Bildung dieses Nebenproduktes weitgehend unterdrücken, wenn man statt der freien Makrocyclen deren Hydrochloride einsetzt und die Reaktion in Gegenwart katalytischer Mengen von Chlorwasserstoff durchführt. Ebenfalls entscheidend ist die Verwendung eines geeigneten dipolaren aprotischen Lösungsmittels. Auf diese Weise läßt sich das gewünschte Produkt mit 75 bis 85 %iger Ausbeute gewinnen. Die Spaltung der Phosphonsäureester erfolgt durch Erhitzen der Substanz in konzentrierter Salzsäure.

Bei dem erfindungsgemäßen Verfahren wird schließlich das cyclische Aminderivat, das am Ende der Seitenkette (R⁵ in Formel (I)), eine funktionelle Gruppe trägt, mit Hilfe dieser funktionellen Gruppe an die zu markierende Substanz gebunden. Gegebenenfalls nach entsprechender Reinigung des Konjugats (z. B. durch Ultrafiltration oder Dialyse bei Proteinen oder Polymeren, durch Säulenchromatographie bei niedermolekularen Substanzen wie Steroiden oder Lipiden) werden Technetium in Form von Pertechnetat bzw. Rhenium in der Form von Perrhenat und ein geeignetes Reduktionsmittel zur Reduktion des Pertechnetats bzw. Perrhenats in die für die Komplexierung benötigte Oxidationsstufe, in beliebiger Reihenfolge oder gemeinsam zugegeben. Das markierte Substrat wird gegebenenfalls nochmals gereinigt. Alternativ kann auch zunächst der Technetium- bzw. Rheniumkomplex des cyclischen Amins erzeugt werden und dann dieser mit der Substanz zum Konjugat umgesetzt werden. Hierbei verläuft die Komplexierung und Reduktion wie oben beschrieben. Vorzugsweise wird die Komplexierungsreaktion bei basischem pH (7 bis 14) durchgeführt.

Die Reduktion des Pertechnetats bzw. des Perrhenats kann nach literaturbekannten Verfahren erfolgen, bevorzugt mit einer Zinn-II-Verbindung. Besonders bevorzugt erfolgt die Reduktion mit einem komplexstabilisierten Zinn-II-Salz, nach einem "Markierungsverfahren", wie dies in der deutschen Offenlegungsschrift DE-A 3728599 vorgeschlagen wurde. Dabei wird zunächst die Zinn-II-Verbindung mit einem Komplexbildner, vorzugsweise einer Phosphorverbindung wie einem Phosphonat oder Pyrophosphat oder Citronensäure versetzt, der dafür sorgt, daß die Zinnverbindung vor allem im physiologischen pH-Bereich in Lösung bleibt. Diese komplexstabilisierte Zinn-II-Salz-Lösung kann dann entweder zu der zu markierenden Substanz gegeben werden, worauf die Zugabe der Pertechnetat- bzw. Perrhenat-Lösung erfolgt oder aber zu einer Mischung aus der zu markierenden Substanz und der Pertechnetat- bzw. Perrhenat-Lösung.

Die vorliegende Erfindung betrifft weiterhin ein Diagnostikum enthaltend einen Chelatkomplex, wie oben definiert, mit Technetium-99m und eine organspezifische Substanz entsprechend obiger Definition.

Darüberhinaus betrifft die vorliegende Erfindung ein Therapeutikum enthaltend einen Chelatkomplex entsprechend obiger Definition mit Rhenium-186 oder -188 und eine organspezifische Substanz, wie oben angegeben.

Ferner betrifft die vorliegende Erfindung einen Testkit zum Nachweis von Tumoren, bestehend aus zwei getrennten, lyophilisierten Komponenten, von denen eine einen Antikörper, der gegen Tumor-assoziierte Antigene gerichtet ist, oder dessen F(ab')₂-Fragment, das mit einem makrozyklischen Amin der Formel (I) nach Anspruch 1 verknüpft ist, und die andere ein Reduktionsmittel enthält, das zur Reduktion und Bindung des Technetiums an der Antikörperkomponente benötigt wird.

In einer bevorzugten Ausführungsform wird ein Zinn-II-Salz als Reduktionsmittel verwendet. Besonders bevorzugt wird dabei das Zinn-II-Salz der Methandiphosphonsäure, Aminomethandiphosphonsäure, 3,3-Diphosphonopropionsäure, 3,3-Diphosphono-1,2-propandicarbonsäure, Citronensäure oder der Propan-1,1,3,3-tetraphosphonsäure eingesetzt.

Im folgenden wird die Erfindung anhand eines Beispiels näher erläutert und in den Patentansprüchen definiert.

### Beispiel

### Darstellung von 1-[6-(2-Vinyl-6-hexyloxymethyl)-pyridin]-4,8,11-trimethylphosphonsäurediethyl-1,4,8,11-tetraazacyclotetradecan:

### 1) 2,6-Pyridindimethanolmonotritylether:

50 g 2,6-Pyridindimethanol werden in 200 ml Pyridin gelöst und mit 0,5 g Dimethylaminopyridin versetzt. 50 g Tritylchlorid werden portionsweise bei Raumtemperatur zugesetzt. Nach einer Stunde erwärmt man zur Vervollständigung der Reaktion für 30 Minuten auf 60°C. Nach Entfernen des Lösungsmittels nimmt man in 200 bis 300 ml Methanol auf und filtriert vom Rückstand ab. Nach Einengen wird der Rückstand in Dichlormethan aufgenommen und vom unlöslichen Rückstand abfiltriert. Das im Filtrat enthaltene Rohprodukt wird durch Chromatographie an Kieselgel gereinigt (Eluent: Dichlormethan/Essigsäureethylester, Gradient von 100/0 bis 80/20). Ausbeute: 51 g (74 %).
¹H-NMR (CDCl₃) [ppm]: 7,8 bis 7,0 (Trityl-H, Pyridin-H), 4,7 (Benzyl-H), 4,3 (Benzyl-H, 3,7 bis 3,6 (OH).

### 2) 6-(Trityloxymethyl)-pyridin-2-al:

9,6 g 2,6-Pyridindimethanolmonotritylether werden in 100 ml Dichlormethan gelöst und über mehrere Stunden portionsweise mit einem Überschuß an Mangandioxid versetzt. Nach beendeter Reaktion werden die Manganrückstände abfiltriert und die Lösung eingeengt.
Das Rohprodukt (9,2 g) wird ohne weitere Reinigung in der nächsten Reaktion umgesetzt.

### 3) 2-Vinyl-6-(hydroxymethyl)-pyridin:

9,2 g 6-(Trityloxymethyl)-pyridin-2-al werden in 150 ml trockenem THF vorgelegt und portionsweise bis zur vollständigen Reaktion mit Methyltriphenylphosphoniumbromid/Natriumamid versetzt. Nach Verdünnen mit Diisopropylether wird der Niederschlag abgetrennt und das Lösungsmittel abgedampft.
Das Rohprodukt wird in 50 ml Dichlormethan aufgenommen und mit 100 ml Salzsäure 15 Minuten gerührt. Die organischen Rückstände werden mit Dichlormethan exthrahiert. Anschließend stellt man die wässrige Phase alkalisch und exthrahiert das Reaktionsprodukt mit Dichlormethan. Die Reinigung erfolgt an Kieselgel (Eluent: Dichlormethan/Essigsäureethylester, Gradient von 100/0 bis 80/20). Gesamtausbeute: 3,8 g (55 %).
¹H-NMR (CDCl₃) [ppm]: 7,8 bis 7,0 (Pyridin-H), 6,8 (Vinyl-H), 6,15 (Vinyl-H), 5,55 (Vinyl-H), 4,8 (Benzyl-H), 4,1 (OH).

### 4) 2-Vinyl-6-(3-brompropyloxymethyl)-pyridin:

150 mg 2-Vinyl-6-(hydroxymethyl)-pyridin werden in 25 ml THF gelöst, mit 0,8 ml Dibromhexan und 70 mg Kaliumhydroxid versetzt. Das nach vollständiger Reaktion und wässriger Aufarbeitung erhaltene Rohprodukt wird säulenchromatographisch gereinigt (Eluent: Dichlormethan/Essigsäureethylester, Gradient von 100/0 bis 80/20). Ausbeute: 155 mg (73 %).
¹H-NMR (CDCl₃) [ppm]: 7,8 bis 7,3 (Pyridin-H), 6,8 (Vinyl-H), 6,15 (Vinyl-H), 5,55 (Vinyl-H), 3,7 bis 3,3 (CH₂Br, CH₂O), 2,2 (CH₂).

### 5) 2-Vinyl-6-(hydroxymethyl)-pyridin:

150 mg 2-Vinyl-6-(hydroxymethyl)-pyridin werden in 25 ml THF gelöst, mit 1 ml Dibromhexan und 70 mg Kaliumhydroxid versetzt und bis zur vollständigen Reaktion bei Raumtemperatur gerührt. Das nach wässriger Aufarbeitung erhaltene Rohprodukt wird Säulenchromatographisch gereinigt (Eluent: Dichlormethan/Essigsäureethylester, Gradient von 100/0 bis 80/20). Ausbeute: 170 mg (75 %).
¹H-NMR (CDCl₃) [ppm]: 7,8 bis 7,2 (Pyridin-H), 6,8 (Vinyl-H), 6,15 (Vinyl-H), 5,5 (Vinyl-H), 4,7 (Benzyl-H), 3,7 bis 3,3 (CH₂Br, CH₂O), 1,9 bis 1,3 (aliphatische H).

### 6) 2-Vinyl-6-(12-Bromdodecyloxymethyl)-pyridin:

150 mg 2-Vinyl-6-(hydroxymethyl)-pyridin werden in 25 ml THF gelöst, mit 1,3 g Dibromdecan und 600 mg Kaliumcarbonat versetzt. Das nach vollständiger Reaktion und wässriger Aufarbeitung erhaltene Rohprodukt wird säulenchromatographisch gereinigt (Eluent: Dichlormethan/Essigsäureethylester, Gradient von 100/0 bis 80/20). Ausbeute: 280 mg (77 %).
¹H-NMR (CDCl₃) [ppm]: 7,8 bis 7,2 (Pyridin-H), 6,8 (Vinyl-H), 6,15 (Vinyl-H), 5,5 (Vinyl-H), 4,7 (Benzyl-H), 3,7 bis 3,3 (CH₂Br, CH₂O), 1,9 bis 1,2 (aliphatische H).

### 7) 1-[3-(2-Vinyl-6-propyloxymethyl)-pyridin]-1,4,8,11-tetraazacyclotetradecan:

2,6 g 1,4,8,11-Tetraazacyclotetradecan werden in 100 ml siedendem Dioxan gelöst und mit 513 mg Kaliumhydroxid sowie 1,3 g 2-Vinyl-6-(6-brompropyloxymethyl)-pyridin versetzt. Anschließend erhitzt man bis zur vollständigen Reaktion am Rückfluß. Nach Verdampfen des Losungsmittels wird das Rohprodukt an Kieselgel chromatographiert (Eluent: Methanol/Ammoniak, Gradient von 100/0 bis 80/20). Ausbeute: 1,3 g (72 %).
¹H-NMR (CDCl₃) [ppm]: 7,8 bis 7,2 (Pyridin-H), 6,8 (Vinyl-H), 6,15 (Vinyl-H), 5,5 (Vinyl-H), 4,7 (Benzyl-H), 3,7 (OCH₂), 2,8 bis 2,2 (NCH₂), 1,9 bis 1,6 (-CH₂-).

### 8) 1-[6-(2-Vinyl-6-hexyloxymethyl)pyridin]-1,4,8,11-tetrazacyclotetradecan:

2,6 g 1,4,8,11-Tetraazacyclotetradecan werden in 100 ml siedendem Dioxan gelöst und mit 513 mg Kaliumhydroxid sowie 1,3 g 2-Vinyl-6-(6-brompropyloxymethyl)-pyridin versetzt. Anschließend erhitzt man bis zur vollständigen Reaktion am Rückfluß. Nach Verdampfen des Lösungsmittels wird das Rohprodukt an Kieselgel chromatographiert (Eluent: Methanol/Ammoniak, Gradient von 100/0 bis 80/20). Ausbeute: 1,5 g (75 %).
¹H-NMR (CDCl₃) [ppm]: 7,8 bis 7,2 (Pyridin-H), 6,8 (Vinyl-H), 6,15 (Vinyl-H), 5,5 (Vinyl-H), 4,7 (Benzyl-H), 3,6 (OCH₂), 2,8 bis 2,2 (NCH₂), 1,8 bis 1,2 (-CH₂-).

### 9) 1-[12-(Dodecyoxymethyl)-pyridin-2-al)-1,4,8,11-tetraazacyclotetradecan:

2,6 g 1,4,8,11-Tetraazacyclotetradecan werden in 100 ml siedendem Dioxan gelöst und mit 513 mg Kaliumhydroxid sowie 1,3 g 2-Vinyl-6-(12-bromdodecyloxymethyl)-pyridin versetzt. Anschließend erhitzt man bis zur vollständigen Reaktion am Rückfluß. Nach Verdampfen des Lösungsmittels wird das Rohprodukt an Kieselgel chromatographiert (Eluent: Methanol/Ammoniak, Gradient von 100/0 bis 80/20). Ausbeute: 1,2 g (69 %).
¹H-NMR (CDCl₃) [ppm]: 7,8 bis 7,2 (Pyridin-H), 6,8 (Vinyl-H), 6,15 (Vinyl-H), 5,5 (Vinyl-H), 4,7 (Benzyl-H), 3,6 (OCH₂), 2,8 bis 2,3 (NCH₂), 1,8 bis 1,2 (-CH₂-).

### 10) 1-[6-(2-Vinyl-6-hexyloxymethyl)-pyridin]-4,8,11-trimethylphosphonsäurediethyl-1,4,8,11-tetraazacyclotetradecan:

150 mg 1-[6-(2-Vinyl-6-hexyloxymethyl)-pyridin]-1,4,8,11-tetraazacyclotetradecan werden in das entsprechende Hydrochlorid überführt, in 20 ml Acetonitril suspendiert und in Gegenwart von pulverisiertem Molsieb unter Schutzgas mit überschüssigem Para-Formaldehyd und Phoshorigsäurediethylester in Gegenwart katalytischer Mengen von Chlorwasserstoff bis zur vollständigen Reaktion am Rückfluß erhitzt. Nach wässriger Aufarbeitung wird das Rohprodukt an Kieselgel chromatographiert (Eluent: Methanol/Ammoniak, Gradient von 100/0 bis 50/50). Ausbeute: 210 mg (79 %).
¹H-NMR (CDCl₃): 7,8 bis 7,2 (Pyridin-H), 6,8 (Vinyl-H), 6,15 (Vinyl-H), 5,5 (Vinyl-H), 4,7 (Benzyl-H), 4,1 (POCH₂), 3,6 (OCH₂), 2,9 (PCH₂), 2,8 bis 2,3 (NCH₂), 1,8 bis 1,2 (-CH₂-), 1,3 (POCH₂CH₃).

### 11) 1-[6-(2-Vinyl-6-hexyloxymethyl)-pyridin]-4,8,11-trimethylphosphonsäurediethyl-1,4,8,11-tetraazacyclotetradecan:

100 mg 1-[6-(2-Vinyl-6-hexyloxymethyl)-pyridin]-4,8,11-trimethylphosphonsäurediethyl-1,4,8,11-tetraazacyclotetradecan werden in 5 ml konzentrierter Salzsäure am Rückfluß erhitzt. Ausbeute: 90 mg (95 %)
¹H-NMR (CDCl₃) [ppm]: 8,4 bis 7,6 (Pyridin-H), 6,8 (Vinyl-H), 6,3 (Vinyl-H), 5,9 (Vinyl-H), 4,7 (Benzyl-H), 3,6 (OCH₂), 3,2 (PCH₂), 3,5 bis 3,0 (NCH₂), 2,1 bis 1,2 (-CH₂-).

## Patentansprüche

1. Verbindungen gemäß Formel (I), 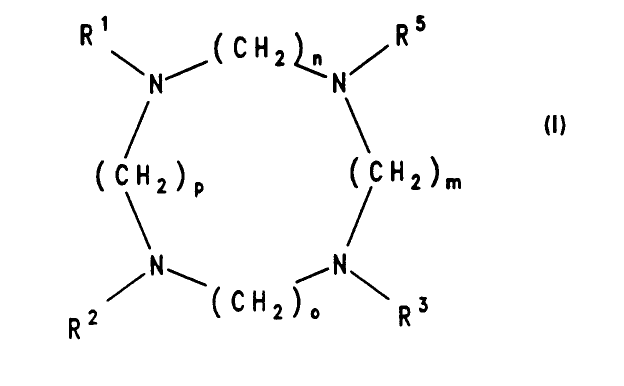 worin
R¹, R² und R³ gleich oder verschieden sind und Wasserstoff oder -CH₂-PO₂HR⁴ und
m, n, o, p gleich oder verschieden sind und 1, 2, 3 oder 4 bedeuten und
R⁴ Hydroxy, Alkoxy mit 1 bis 4 C-Atomen oder Phenoxy, welches gegebenenfalls mit C₁- bis C₄-Alkyl substituiert ist und
R⁵ eine Seitenkette der Formel (II) ist,
[-X-(Y)_{q}]ᵣ-(Z)ₛ-R⁶ (II)
wobei
q für 0 oder 1 und
r für 1, 2, 3, 4 oder 5 und
s für 0 oder 1 steht, mit der Maßgabe, daß S nur 1 sein kann, wenn
q = 1
und
X, Z gleich oder verschieden sind und Alkylen mit 1 bis 40 C-Atomen, ortho-, meta- oder para-Aralkylen mit 7 bis 40 C-Atomen, welches über den Alkylrest am Stickstoff und über den Arylrest oder einen weiteren am Arylrest gebundenen Alkylrest an R⁶ gebunden ist, bedeuten und
Y einen Substituenten einer der folgenden Formeln bedeutet, 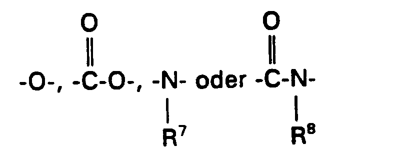 wobei
R⁷ Wasserstoff, Methyl, Ethyl, Benzyl, Acetyl oder Benzoyl und
R⁸ Wasserstoff, Methyl, Ethyl oder Benzyl bedeuten und
R⁶ eine Gruppe einer der folgenden Formeln bedeutet 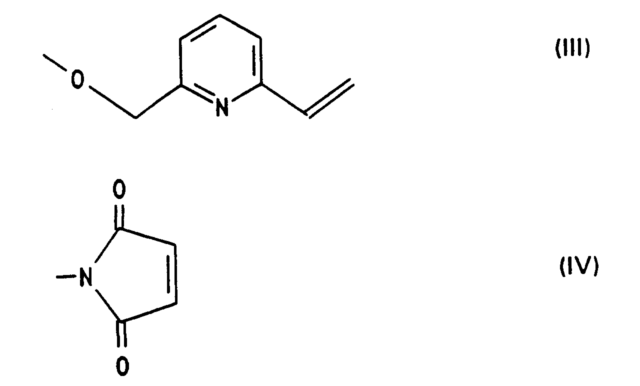
-N=C=S (V) 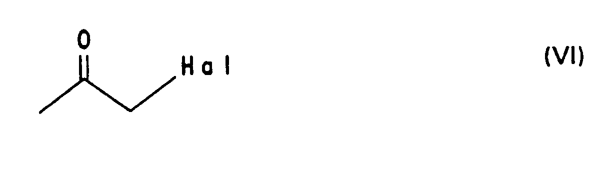
oder
-N₃, Hal oder -SH,
wobei Hal für Fluor, Chlor, Brom oder Iod steht.

2. Verbindungen gemäß Anspruch 1, dadurch gekennzeichnet, daß
R¹, R² und R³ gleich sind und eine Verbindung der Formel -CH₂-PO₂HR⁴ bedeuten und
m, n, o, p gleich oder verschieden sind und 2 oder 3 bedeuten und
X, Z gleich oder verschieden sind und Alkylen mit 1 bis 20 C-Atomen, ortho-, meta- oder para-Aralkylen mit 7 bis 20 C-Atomen, welches über den Alkylrest am Stickstoff und über den Arylrest oder einen weiteren am Arylrest gebundenen Alkylrest an R⁶ gebunden ist, bedeuten.

3. Verbindungen gemäß Anspruch 1 oder 2, dadurch gekennzeichnet, daß
Y einen Substituenten einer der folgenden Formel bedeutet 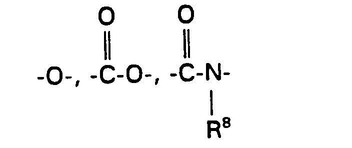 worin
R⁸ für Wasserstoff, Methyl, Ethyl oder Benzyl steht und
R⁶ eine Gruppe einer der folgenden Formeln bedeutet 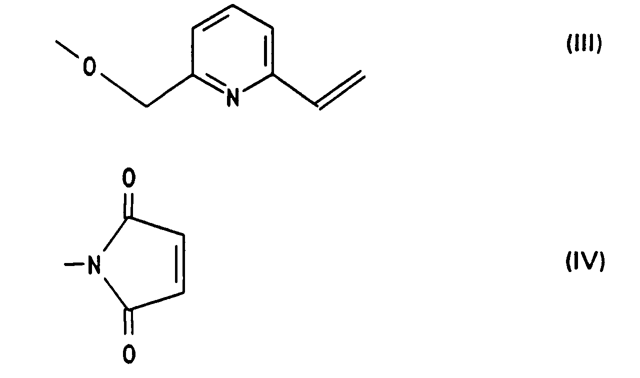
-N=C=S (V) 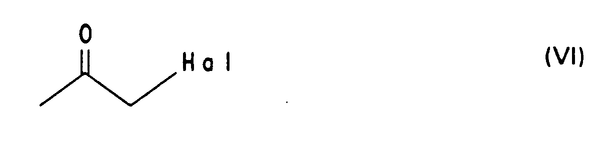
wobei Hal für Fluor, Chlor, Brom oder Iod steht
oder -N₃.

4. Verbindungen nach einem oder mehreren der Ansprüche 1 bis 3, dadurch gekennzeichnet, daß
Y für -O-und
R⁶ für eine Gruppe einer der folgenden Formeln steht

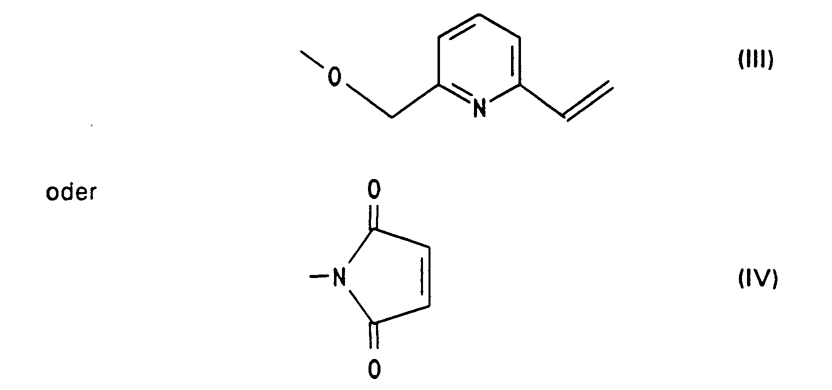

5. Verbindungen nach einem oder mehreren der Ansprüche 1 bis 4, dadurch gekennzeichnet, daß
R⁶ für eine Gruppe der folgenden Formel steht

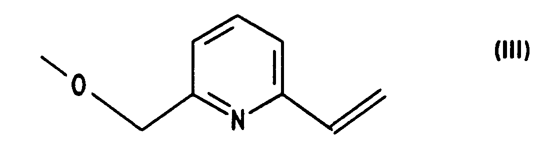

6. Eine Verbindung der Formel (VII)

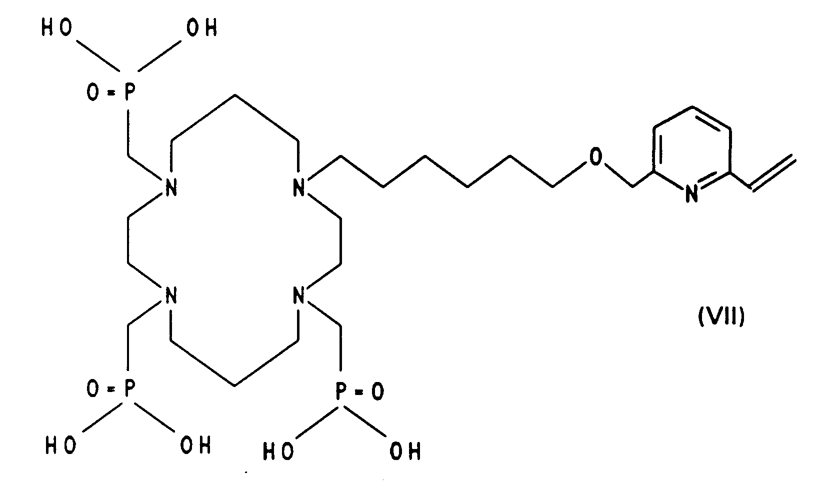

7. Chelatkomplex, enthaltend eine Verbindung der Formel (I) gemäß Anspruch 1 und ein Technetium- oder Rheniumion.

8. Konjugat enthaltend einen Chelatkomplex gemäß Anspruch 7 und, über die Seitenkette R⁵ des Chelatbildners gebunden, eine Substanz von diagnostischem und/oder therapeutischem Interesse.

9. Konjugat nach Anspruch 8, dadurch gekennzeichnet, daß die Substanz von diagnostischem und/oder therapeutischem Interesse eine hohe Organspezifität besitzt.

10. Konjugat nach Anspruch 8, dadurch gekennzeichnet, daß die Substanz von diagnostischem und/oder therapeutischem Interesse aus einem Antikörper oder aus Antikörperfragmenten besteht.

11. Konjugat nach Anspruch 8, dadurch gekennzeichnet, daß die Substanz von diagnostischem und/oder therapeutischem Interesse ein Oligonukleotid oder ein Peptid ist.

12. Verfahren zur Herstellung von Verbindungen der Formel (I) gemäß Anspruch 1, dadurch gekennzeichnet. daß
a) ein Azamakrozyklus in Gegenwart basischer Mittel mit einem Alkylhalogenid, enthaltend eine Kopplungsgruppe für Substanzen von diagnostischem und/oder therapeutischem Interesse, umgesetzt wird oder
b) ein Azamakrozyklus in Gegenwart basischer Mittel mit einem Alkylhalogenid umgesetzt wird und daß in einem weiteren Verfahrensschritt eine Kopplungsgruppe für Substanzen von diagnostischem und/oder therapeutischem Interesse eingeführt wird.

13. Verfahren zur Markierung von Substanzen von diagnostischem oder therapeutischem Interesse mit Technetium- bzw. Rheniumionen, dadurch gekennzeichnet. daß
a) die zu markierende Substanz mit einer Verbindung der Formel (I) gemäß Anspruch 1 umgesetzt wird und die so erhaltene Verbindung mit Technetium- oder Rheniumisotopen markiert wird, indem man sie mit Pertechnetat oder Perrhenat und einem Reduktionsmittel für Pertechnetat oder Perrhenat versetzt oder daß man
b) zunächst den Technetium- oder Rheniumkomplex durch Umsetzung einer Verbindung der Formel (I) gemäß Anspruch 1 mit Pertechnetat oder Perrhenat und einem Reduktionsmittel für Pertechnetat oder Perrhenat herstellt und anschließend diesen Technetium- oder Rheniumkomplex mit der zu markierenden Substanz umsetzt.

14. Diagnostikum enthaltend einen Chelatkomplex gemäß Anspruch 7 mit Technetium-99m und eine organspezifische Substanz.

15. Therapeutikum, enthaltend einen Chelatkomplex gemäß Anspruch 7 mit Rhenium-186 oder -188 und eine organspezifische Substanz.

16. Testkit zum Nachweis von Tumoren, bestehend aus zwei getrennten, lyophilisierten Komponenten, von denen eine einen Antikörper, der gegen Tumor-assoziierte Antigene gerichtet ist, oder dessen F(ab')₂-Fragment im Gemisch mit einem Puffer, wobei der Antikörper oder dessen Fragment mit einem makrozyklischen Amin der Formel (I) nach Anspruch 1 verknüpft ist, und die andere ein Reduktionsmittel enthält, das zur Reduktion und Bindung des Technetiums an der Antikörperkomponente benötigt wird.

17. Testkit nach Anspruch 16, dadurch gekennzeichnet, daß das Reduktionsmittel ein Zinn-II-Salz ist.

18. Testkit nach Anspruch 17, dadurch gekennzeichnet, daß das Zinn-II-salz der Methandiphosphonsäure, Aminomethandiphosphonsäure, 3,3-Diphosphonopropionsäure, 3,3-Diphosphono-1,2-propandicarbonsäure, Citronensäure oder der Propan-1,1,3,3-tetraphosphonsäure eingesetzt wird.
